Europäisches Patentamt

European Patent Office

Office européen des brevets

(11) Publication number: **0 244 920 B1**

# EUROPEAN PATENT SPECIFICATION

(45) Date of publication of patent specification: **11.12.91**   (51) Int. Cl.⁵: **C12N 9/04**, C12N 15/53, C11D 3/386, C12Q 1/26

(21) Application number: **87201039.2**

(22) Date of filing: **02.06.87**

(54) **Process for preparing a catalase-free oxidase and a catalase-free oxidase-containing yeast, and use thereof.**

(30) Priority: **05.06.86 NL 8601454**

(43) Date of publication of application:
**11.11.87 Bulletin 87/46**

(45) Publication of the grant of the patent:
**11.12.91 Bulletin 91/50**

(84) Designated Contracting States:
**AT BE CH DE ES FR GB GR IT LI NL SE**

(56) References cited:
**EP-A- 0 019 937**
**EP-A- 0 071 990**
**EP-A- 0 173 378**
**FR-A- 2 218 346**
**US-E- 32 016**

**CHEMICAL ABSTRACTS, vol. 93, no. 21, 24th November 1980, page 348, abstract no. 200741z, Columbus, Ohio, US; L. EGGELING et al.: "Regulation of alcohol oxidase synthesis in Hansenula polymorpha: oversynthesis during growth on mixed substrates and induction by methanol", & ARCH. MICROBIOL. 1980, 127(2), 119-24**

(73) Proprietor: **UNILEVER NV**
**Burgemeester s'Jacobplein 1 P.O. Box 760**
**NL-3000 DK Rotterdam(NL)**

(84) Designated Contracting States:
**BE CH DE ES FR GR IT LI NL SE AT**

Proprietor: **UNILEVER PLC**
**Unilever House Blackfriars P.O. Box 68**
**London EC4P 4BQ(GB)**

(84) Designated Contracting States:
**GB**

(72) Inventor: **Giuseppin, Marco Luigi Federico**
**Munterstraat 13**
**NL-3123 PN Schiedam(NL)**
Inventor: **Van Eijk, Hendrikus Marius Johannes**
**Floreslaan 150**
**NL-3131 NE Vlaardingen(NL)**
Inventor: **Van Dijken, Jan Pieter**
**Geerbron 18**
**NL-2641 LL Pijnacker(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 96, no. 9, 1st March 1982, page 301, abstract no. 65423s, Columbus, Chio, US; L. EGGELING et al.: "Enhanced utilization-rate of methanol during growth on a mixed substrate: a continuous culture study with Hansenula polymorpha", & ARCH. MICROBIOL. 1981, 130(5), 362-5

CHEMICAL ABSTRACTS, vol. 88, no. 25, 19th June 1978, page 287, abstract no. 185105w, Columbus, Ohio, US; G.T. MIWA et al.: "The direct oxidation of ethanol by a catalase- and alcohol dehydrogenase-free reconstituted system containing cytochrome P-450", & ARCH. BIOCHEM. BIOPHYS. 1978, 187(2), 464-75

MICROBIOLOGY ABSTRACTS: SECTION A. INDUSTRIAL & APPLIED MICROBIOLOGY, vol. 17, no. 4, 1982, page 7, abstract no. 2547-A17, Oxford, GB; R.N. PATEL et al.: "Microbial oxidation of methanol: properties of crystallized alcohol oxidase from a yeast", & ARCH. BIOCHEM. BIOPHYS., vol. 210, no. 2, September 1981, pages 481-488

CHEMICAL ABSTRACTS, vol. 87, no. 25, 19th December 1977, page 537, abstract no. 199125u, Columbus, Ohio, US; V.S. PODGORSKII et al.: "Production of protein-vitamin concentrates from methyl alcohol", & REF. NAUCHNO-ISSLED. RAB. INST. MIKROBIOL. VIRUSOL. AKAD. NAUK UKR. SSR 1974 (Pub. 1976), 17-19

(74) Representative: **van der Toorren, Johannes, Drs. et al**
**UNILEVER N.V. Patent Division Postbus 137 NL-3130 AC Vlaardingen(NL)**

## Description

The invention relates to a process for the preparation of an oxidase by aerobic fermentation of a yeast under conditions in which the yeast produces oxidase, and, if desired, isolation from the yeast cells of the oxidase produced.

Oxidases are enzymes which catalyse specific oxidation reactions of certain substrates and thereby produce $H_2O_2$. An example of an oxidase is methanol oxidase (MOX) that enables methylotrophic yeasts such as Hansenula polymorpha, Candida boidinii, Pichia pastoris and suchlike to live on methanol. The MOX catalyses the oxidation of methanol to formaldehyde and hydrogen peroxide, during which molecular oxygen acts as electron acceptor. The formaldehyde formed is used for assimilation (anabolism), during which cell material is produced via the xylulose monophosphate route, or used for dissimilation (catabolism), during which it is converted into formic acid and finally into carbon dioxide. With these further processes other enzymes play an important rôle: in the assimilation process dihydroxyacetone synthetase is important, in the dissimilation process formaldehyde dehydrogenase and formiate dehydrogenase are key enzymes. The hydrogen peroxide formed by the oxidase is extremely toxic for these organisms and, according to the present state of the art, is immediately rendered innocuous by another enzyme, namely catalase.

Other examples of oxidases are other alcohol oxidases, glucose oxidase, glycerol oxidase, D-aminoacid oxidase, aldehyde oxidase, amine oxidase, arylalcohol oxidase, galactose oxidase, sorbose oxidase, uric acid oxidase and xanthine oxidase.

Oxidases can be used for various purposes. An important use lies in the field of washing and bleaching compositions, where the presence of oxidase during the washing or bleaching process can lead to the in situ formation of hydrogen peroxide if at the same time a suitable substrate for the oxidase is present. The hydrogen peroxide displays a bleaching agent activity, or can, in co-operation with bleaching agent precursors such as TAED (tetraacetyl ethylene diamine), lead to the formation of bleaching agents which are active at low temperatures, such as peracids. An important advantage of this system, in which the washing or bleaching agent itself contains no hydrogen peroxide but only the enzyme that during the washing or bleaching process catalyses the formation of hydrogen peroxide in situ, is that inactivation of washing composition components, such as proteases, lipases and suchlike, during storage and transport is prevented. This use of oxidases is described in the British patent publications 1 225 713 and 2 101 167 and in the German patent publication 2 557 623.

Another use of oxidases lies in the field of qualitative and/or quantitative analysis, e.g. for determining the alcohol content of systems which are to be investigated, such as fermentation liquids. The analysis is based on a measurement of the hydrogen peroxide formed via an oxidation of colouring substances catalysed by peroxidase.

Oxidases can also be used within the framework of chemical syntheses or of processes for the purification of refuse in order to catalyse oxidation of substrates.

For the most of these uses, and certainly for that as component of washing or bleaching compositions and for that as aid in analysis, it is of great importance that the oxidase is free of catalase, which would decompose the hydrogen peroxide formed. A well-known problem with the microbiologically produced oxidases such as methanol oxidase (EC 1.1.3.13) by fermentation of methylotrophic yeasts such as Hansenula polymorpha, Candida boidinii, Pichia pastoris and such-like on methanol, is, however, that they are constantly accompanied by the natural catalase enzyme [Veenhuis, M., van Dijken, J.P. and Harder, W. (1983) in Advances in Microbial Physiology, Rose, A.H., Gareth Morris, J. and Tempest, D.W., Editors, Vol. 24, pp 1-82, Academic Press, New York] that almost immediately decomposes the peroxide formed, so that no effective bleaching agent activity or accurate analysis of the substrate concentration is possible. It is true that methods are known (see for example the British patent specification 2 101 167) for removing the catalase from the product to a great extent or for inactivating it and thus obtaining an active, substantially catalase-free oxidase, but for technical application these methods (such as catalase removal through ion exchange chromatography or gel filtration, and catalase inactivation through treatment with sodium dodecylsulphate (SDS) or sodium azide, and so on) are too expensive and cumbersome and in many cases inadequate, or, in the case of sodium azide, a toxic material is even introduced.

Consequently there is need of a method with which catalase-free oxidase can be obtained in a technically and economically justifiable manner. A microbiological production, in which catalase-negative mutants are used, could meet this need, but seems impracticable because, on the basis of the literature, it could be expected that the organisms without catalase are doomed to die if cultured in a nutritive medium to which substrate for the oxidase has been added in behalf of a good growth of the micro-organism and strong expression of the oxidase gene.

In the case of methanol oxidase (MOX) it is known that the formation of MOX, formiate dehydrogenase, formaldehyde dehydrogenase, dihydroxyacetone synthetase and catalase is subject to glucose repression, as well as to glycerol and mannitol repression. Derepression of MOX synthesis occurs if the organisms are cultured at very low glucose, glycerol or mannitol concentrations, but the MOX production is then still relatively very small.

For a good understanding of the previous and the following paragraphs, some definitions are appropriate. An inductor is a compound that promotes the transcription of a gene by an interaction with the promotor, by inactivation of the effective repressor or by blocking of a repressor gene.

A repressor is a compound that blocks the transcription of a gene by an interaction with the promoter or by an activation of a repressor protein gene.

Synthesis of a gene product (transcription of a gene) can occur by effects of an inducer (induction) or by the relief of the repressor mediated repression (derepression).

In the case of MOX, methanol acts as an inducer in both batch and continuous cultures. Glycerol, sorbitol, glucose and ethanol are known repressors in batch and continuous cultures.

Under conditions with low concentrations of glycerol, sorbitol and glucose in batch cultures and in steady state continuous cultures at low dilution rates, a derepression of MOX occurs.

It is known that complete induction of the MOX synthesis only takes place when the organisms are cultured on methanol as only source of carbon or on specific mixtures of methanol with glucose, glycerol or mannitol [see Eggeling and Sahm, Arch. Microbiol. 127, (1980) 119-124 and Arch. Microbiol. 130, (1981) 362-365]. These authors conclude from their experiments on the yeast Hansenula polymorpha that methanol itself, and not a metabolite thereof, is responsible for induction of MOX synthesis. From these publications it can be gathered that the level of expression in the situation of repression or derepression is low compared with the level of expression in the situation of induction: in the order of magnitude of 1-2% under complete glucose repression, and about 10% under glucose or glycerol derepression at low growth speeds in continuous cultures. Indeed, in one of these publications [Arch. Microbiol. 127, (1980) 119-124] a catalase-negative mutant of Hansenula polymorpha is described, indicated as mutant 55/11, which mutant cannot grow on methanol, but produces methanol oxidase when grown on glycerol under conditions of derepression. In batch cultures the MOX production of the catalase-negative mutant was about 50% of the amount which is produced on glycerol by the wild-type, i.e. also catalase-producing, strain. Compared with the MOX production of the wild-type strain on methanol, the MOX production of the catalase-negative mutant was only about 17%. Such a relatively small productivity of the preparation of catalase-free oxidase is inadequate for production on a technical scale.

In addition to the literature mentioned above, some other less relevant publications are worth being mentioned.

In Chemical Abstracts 88 (1978) 185105w, referring to G.T. Miwa et al., Arch. Biochem. Biophys. 187 - (1978) 464-475, the direct oxidation of ethanol by a catalase-and alcohol dehydrogenase-free reconstituted system containing cytochrome P-450 and NADPH is described. In view of the required presence of NADPH, this system will not work as an oxidase like MOX capable of oxidizing alcoholic compounds with molecular oxygen, so that a different system is described which does not give any information on or direction to the use of a catalase-negative mutant of a yeast producing an oxidase.

In EP-A- 0 019 937 (PHILLIPS PETROLEUM CO.) a method of producing alcohol oxidase is described, wherein a methanol-utilizing Pichia-type micro-organism, e.g. Pichia pastoris, is used. Also enzyme preparations from a methanol-utilizing micro-organism of genus Pichia, catalyzing the reaction

$$RCH_2OH + O_2 \rightarrow RCHO + H_2O_2$$

wherein R = H, $CH_3$, $C_2H_5$ or $C_3H_7$,
are described, whereby a catalase-free enzyme is produced after several purification treatments. This method suffers from the disadvantages mentioned earlier in this specification. Moreover, the patent publication itself suggests that some catalase is still present in the purified alcohol oxidase.

In EP-A- 0 071 990 (PHILLIPS PETROLEUM CO.) the removal of ambient oxygen from aqueous liquids is described, which removal is catalyzed by enzymatic deoxygenating systems comprising alcohol oxidase in the presence of alcohol optionally with catalase. Although yeasts of the genus Hansenula and the genus Pichia can be used, the use of a catalase-negative mutant according to the present invention has neither been described nor suggested. On the contrary, the presence of catalase is allowed, thus the nature and the purpose of the enzyme preparations used are different from those according to the present invention.

Now it has been found, surprisingly, that a microbiological production of methanol oxidase that is free of catalase can be realized on a technical scale with a process of the kind mentioned in the preamble, when a catalase-negative mutant of the yeast Hansenula polymorpha is allowed to grow in a nutritive medium suitable for the yeast in the presence of at least one compound which induces the expression of the oxidase gene but is not a substrate for the oxidase.

The invention, however, is not limited to the production of methanol oxidase. With the aid of genetic engineering techniques, yeasts, particularly Hansenula and Pichia, can be obtained which contain another oxidase gene, preferably under control of the strong MOX promoter. Depending on the intended use, a need of such other oxidase, such as D-aminoacid oxidase and such-like, can exist.

Also, instead of the catalase-negative Hansenula polymorpha indicated above, other catalase-negative yeasts can also be used. Depending on the oxidase to be produced, a compound other than formaldehyde or formiate might also be used as a compound which induces the expression of the oxidase gene, but is not a substrate for the oxidase. For example, according to K.B. Zwart and W. Harder, J. Gen. Microbiol. 129 - (1983) 3157-3169, the formation of amine oxidase is induced in a medium containing 0.3% (w/v) glycerol and 5 mM ammonium sulphate, i.e. under ammonium-limiting conditions (cf. Table 2 on page 3161). Consequently, the invention is not limited to the production of methanol oxidase.

Therefore, in a general sense, the invention embraces a process for the preparation of an oxidase or an oxidase-containing mixture or preparation by aerobic fermentation of a yeast under conditions in which the yeast produces oxidase, and, if desired, isolation from the yeast cells of the oxidase produced, wherein a catalase-negative mutant of a yeast is allowed to grow in a nutritive medium suitable for the yeast in the presence of at least one compound which induces the expression of the oxidase gene but is not a substrate for the oxidase.

It is preferred according to the invention that a catalase-negative mutant of a yeast of the genus Hansenula or of the genus Pichia, in particular of the species Hansenula polymorpha or of the species Pichia pastoris be used, such as the catalase-negative mutant Hansenula polymorpha 55/11, ATCC 46059. However, the invention is not limited to the use of this one mutant. Other catalase-negative mutants can be obtained by the introduction of any mutations at random and the subsequent selection of the mutants obtained, or by directed mutation methods, for example such as described inter alia in European Patent Application EP-A- 0 173 378 or by deletion of the catalase gene or by a modification of the catalase gene which inhibit catalase expression.

It is further preferred according to the invention that a yeast be used in which the genetic information for the oxidase stands under control of a strong promoter, as a result of which a large yield of oxidase can be realized. An example hereof is the MOX promoter, in particular the MOX promoter of Hansenula polymorpha CBS 4732. This occurs naturally in the Hansenula polymorpha 55/11, ATCC 46059 yeast described hereinbefore, but with other yeasts this can be achieved by means of known recombinant DNA techniques (cf. EP-A- 0 173 378). The strength of the promoter is apparent from the fact that the MOX content of the wild-type strain of Hansenula polymorpha cultured on methanol is in the order of magnitude of 30% of the cellular protein. Examples of other strong promoters are the dihydroxyacetone synthetase (DAS) promoter of methylotropic yeasts and the amine oxidase promoter of yeasts producing amine oxidase.

It is preferred, according to the invention, that as oxidase gene, preferably a methanol oxidase gene is used, for example a methanol oxidase gene that codes for a methanol oxidase with the same amino acid sequence as the known MOX of Hansenula polymorpha CBS 4732 or a derivative of this sequence, obtained via enzyme engineering or a modification thereof which has no really lesser functionality. In combination with a methanol oxidase, preferably methanol is used as inducing substrate. As well as for methanol, the MOX enzyme also appears to be active for various other substrates, such as for ethanol, n-propanol, n-butanol, n-amylalcohol, and even, although to a slighter degree, for compounds such as methylcellosolve, ethyleneglycol, benzyl alcohol, isopropanol, isoamyl alcohol and propyleneglycol. Some of these compounds, such as ethanol, are eminently suitable for serving as substrate when methanol oxidase is used in washing and bleaching compositions, because they are relatively cheap and toxicologically acceptable.

It is preferred, according to the invention, that either formiate or formaldehyde or both are used as the substance which induces expression of the oxidase gene but is not a substrate for the oxidase.

By formiate is meant both formic acid salts, particularly alkali metal and ammonium salts as well as alkaline earth metal salts, and formic acid itself.

Otherwise than could be expected on the grounds of the literature, these metabolites of methanol appear to be able, in yeast of the genus Hansenula, to give induction of the expression of the gene under the control of the MOX promoter. Such an induction of methanol oxidase by formiate and formaldehyde has actually been observed previously, with batch fermentation of yeast of the species Candida boidinii - (Eggeling et al., FEMS Microbiol. Letters 1 (1977), 205-210), as has also induction of methanol oxidase by formiate with batch fermentation of the yeast Kloeckera sp. N° 2201 (Shimizu et al., Agric. Biol. Chem. 41 - (11), 1977, 2215-2220), but according to Eggeling and Sahm, Arch. Microbiol. 127 (1980), 119-124, with Hansenula polymorpha, only methanol is capable of induction.

In connection with production on technical scale, it is preferred according to the invention that the yeast is cultured in a continuous fermentation. However, batch fermentation or "fed batch" fermentation is not excluded. By "fed batch" is meant a "batch" fermentation in which the substrate is not added in one go at the beginning, but gradually so as to achieve a controllable substrate concentration during the whole fermentation.

During the fermentation, a nutritive medium suitable for the yeast, such as Hansenula or Pichia, is used which must contain a carbon source suitable for this organism. Suitable sources of carbon are known to the expert or can be determined by experiment. Glucose, for example, is suitable, but also other sugars, such as sorbose, xylose, sorbitol and other substances such as glycerol, citric acid and suchlike can be used as carbon source. A suitable nutritive medium is known from Egli et al., Arch. Microbiol. 124 (1980), 115-121.

According to the invention it is important that, during the fermentation, a suitable ratio between the amount of carbon source and the amount of formiate or formaldehyde is established.

A preferred embodiment of the invention is characterized in that the yeast is cultured in the presence of a carbon source suitable for the yeast species chosen and of a formiate, the molar ratio of formiate : carbon source being 0.5:1 to 4.5:1. Preferably, a molar ratio of formiate : carbon source of 2:1 to 3.5:1 is used, most preferably of 2.5:1 to 3.2:1.

Another preferred embodiment of the invention is characterized in that the yeast is cultured in the presence of a carbon source suitable for the yeast species chosen and of formaldehyde, the molar ratio of formaldehyde : carbon source being 0.1:1 to 3:1. Preferably a molar ratio of formaldehyde : carbon source of 0.5:1 to 2.5:1 is used, most preferably of 1:1 to 2:1.

In experiments with such formiate/glucose and formaldehyde/glucose mixtures in continuous fermentations at a dilution rate D of 0.05 to 0.1 hour$^{-1}$ of the catalase-negative mutant Hansenula polymorpha 55/11, MOX yields are achieved which are in the order of magnitude of 30%, respectively 60%, of the MOX yield of the wild-type strain cultured on methanol. These are yields which, also with regard to the absence of catalase, are appropriate for use on technical scale.

When the concentrations of formiate or formaldehyde used are too high, a lower cell yield is obtained and the culture can even be killed. Concentrations of formiate or formaldehyde which are too low give insufficient induction, so that a low yield of oxidase is the result. With growth of the mutant on glucose only, without formiate or formaldehyde, the same low MOX activities are obtained as in the case of the wild-type strain.

If, for the intended use, one is unwilling or unable to use the cultured yeast cells as such, the oxidase accumulated in the yeast cells and particularly in the peroxisomes should be isolated from the cells. To do this, usual methods of cell lysis can be applied, such as the physical breaking of the cells with the aid of glass globules, treatment in a so-called French press or Manton Gaulin homogenizer, ultrasonic treatment, and enzymatic methods, e.g. with zymolyase.

According to the invention it has been found that it is advisable in this connection to work under oxygen-free conditions, for example by working under a nitrogen atmosphere. If the cells were to be broken open in the presence of oxygen, substrates of the oxidase enzyme which possibly may be present in the cell plasma or compartments of the yeast cells could be oxidized in the presence of the oxidase released from the peroxisomes, whereafter the hydrogen peroxide formed in this case breaks off the oxidase among other things.

The invention also relates to an oxidase-containing, catalase-negative mutant of a yeast, obtained by making use of a process according to the invention for preparing such a yeast, as well as to an oxidase or an oxidase-containing composition, obtained by isolation of the oxidase from a yeast according to the invention, possibly followed by processing the oxidase thus isolated to a composition with other components.

Further, the invention relates to the use of an oxidase-containing, catalase-negative mutant of a yeast according to the invention or the use of an oxidase isolated therefrom together with a substrate for the oxidase for the in situ production of hydrogen peroxide in a washing or bleaching process. Particularly when an alcohol oxidase is used, ethanol is preferably used as substrate in such a washing process.

Also a washing or bleaching agent that contains an oxidase-containing, catalase-negative mutant of a yeast according to the invention, or the oxidase isolated therefrom, is an embodiment of the invention.

Further, the invention relates to the use of an oxidase-containing, catalase-negative mutant of a yeast according to the invention or of an oxidase isolated therefrom as catalyst for the oxidation of a substrate for the oxidase within the framework of a chemical synthesis or of a process for purifying refuse or for the qualitative and/or quantitative determination of a substrate for the oxidase.

The invention is further explained in the following experimental part.

## Fermentation conditions and media

Hansenula polymorpha was used as a catalase-negative colony from yeast peptone dextrose agar and pre-cultured for one day at 37°C in a medium according to Egli et al., Arch. Microbiol. 124 (1980), 115-121, with glucose as sole carbon source.

Culturing was carried out continuously in a 3-litre fermenter containing 2 litres of medium. The aeration, pH and agitation were regulated, care being taken that the pressure of dissolved oxygen never came below 25% saturation. The pH and foam formation were controlled with the aid of a concentrated ammonia solution which contained an anti-foaming agent based on silicone oil (Rhodorsil R 425® R ex Rhone Poulenc) in a ratio of 4:1 to 1:1. The pH was adjusted to 5.0 ± 0.05 pH units. The temperature was kept at 37 ± 0.2°C. The streams of medium entering and leaving were measured and adjusted to a desired flow rate with the aid of a peristaltic pump. Stable states of the fermentation were tested by measuring the respiration parameters (the respiration quotient, the rate of carbon dioxide development and the rate of oxygen absorption), and by testing the MOX contents in cell suspensions and cell lysates.

## Analyses

## Determination of MOX and catalase

The MOX activity in cell suspensions and cell-free extracts and the catalase activity were determined according to van Dijken et al., Arch. Microbiol. 111 (1976), 137-144. 1 unit corresponds with 1 micromol methanol used per minute at 37°C in an air-saturated 0.1 M phosphate buffer having a pH of 7.5.

## Protein

Protein was determined according to the method of Lowry et al., J. Biol. Chem. 193 (1951), 265-275. Beef serum albumin was used as standard.

## Biomass

The dry weight of the biomass was determined by drying washed cell suspensions at 100°C to constant weight.

Glucose, methanol, formic acid and formaldehyde were determined with the aid of HPLC and standard enzymatic analyses.

## Breaking of cells

Cell lysates were obtained by ultrasonic treatment or by incubation with zymolyase.

## Ultrasonic treatment

Ultrasonic treatment was carried out at 0°C with the aid of a 0.1 M potassium phosphate solution having a pH of 7.5, which contained 5 ml of washed cell suspension, with 3 g glass beads (average diameter 100μm). The cell suspension was treated 5 times for 1 minute with a Branson cell breaker type B-12 (Branson Sonic Power Company). Between the treatments a cooling period of 1 minute was observed. A power of 70 Watt per 5 ml of solution was introduced, for doing which a microtip was used.

Under anaerobic conditions, nitrogen was passed through the suspension for 15 minutes before and during the ultrasonic treatment.

## Lysis procedure

200 ml of cell suspension with an optical density at 610 nm of OD 15 to 18 in 0.1 M sodium phosphate buffer having a pH of 8.5, which contained 5 mM EDTA, 1 mM dithiotreitol and 10 mg Zymolyase 100T ex Arthrobacter luteus (Seikagaku Kogyo Co.), was gently stirred at 37°C in a bottle in a thermostat. After regular intervals of 10 or 15 minutes, samples were taken and analysed for MOX activity, protein content and optical density at 610 nm.

## Example 1

### Production of MOX on formic acid/glucose mixtures

Hansenula polymorpha ATCC 46059 was grown on medium I, described in Table A, which medium contained various ratios of formic acid to glucose.

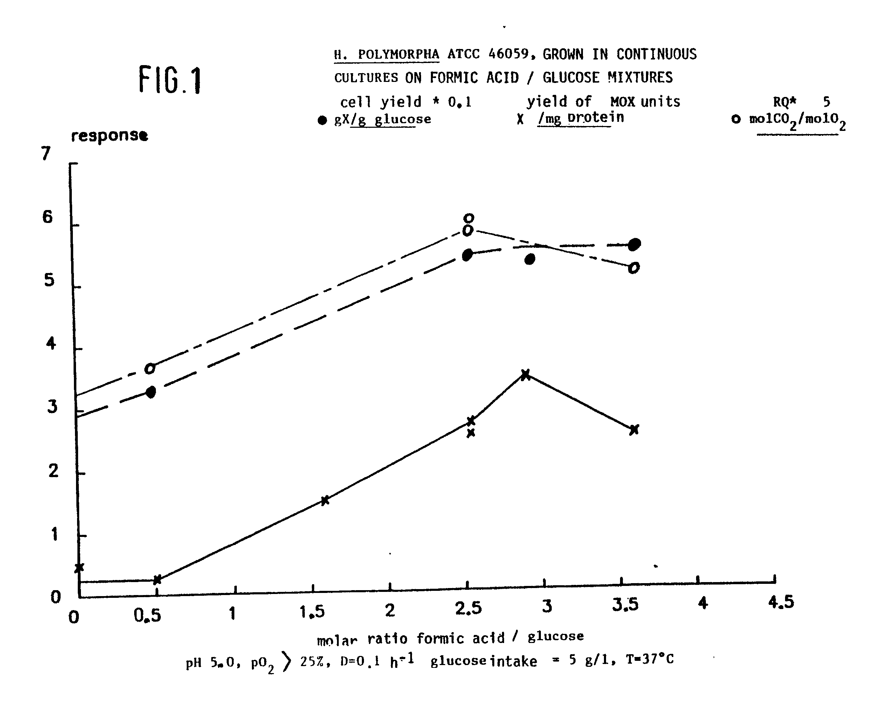

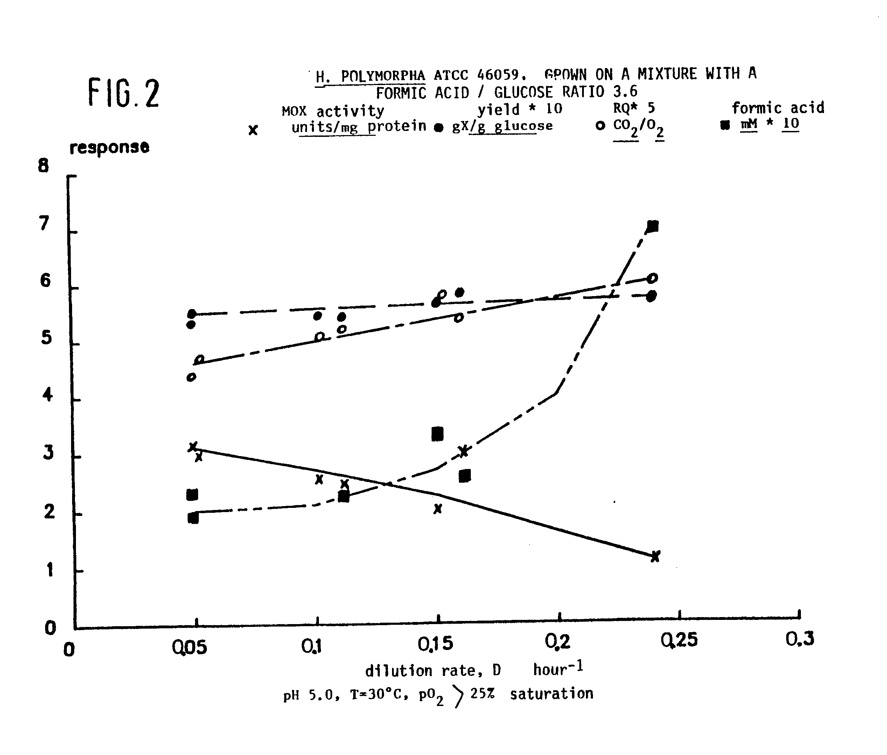

The Figures 1 and 2 and the Tables C and D show the specific MOX activity in the lysates. At a constant dilution rate of 0.1 hour$^{-1}$ an optimal ratio of 2.9 mol formic acid to 1 mol glucose was found.

With a ratio of 3.6 mol formic acid per mol glucose, an optimal dilution rate of 0.05 to 0.15 hour$^{-1}$ was found (Figure 2). Above the optimal ratios (above 2.9) and the optimal dilution rate (above 0.2) a significant content of residual formic acid was found, that is toxic for the culture.

In the cell lysates obtained from the above-mentioned cultures, no catalase activity could be detected.

## Example 2

### Production of MOX on formaldehyde/glucose mixtures

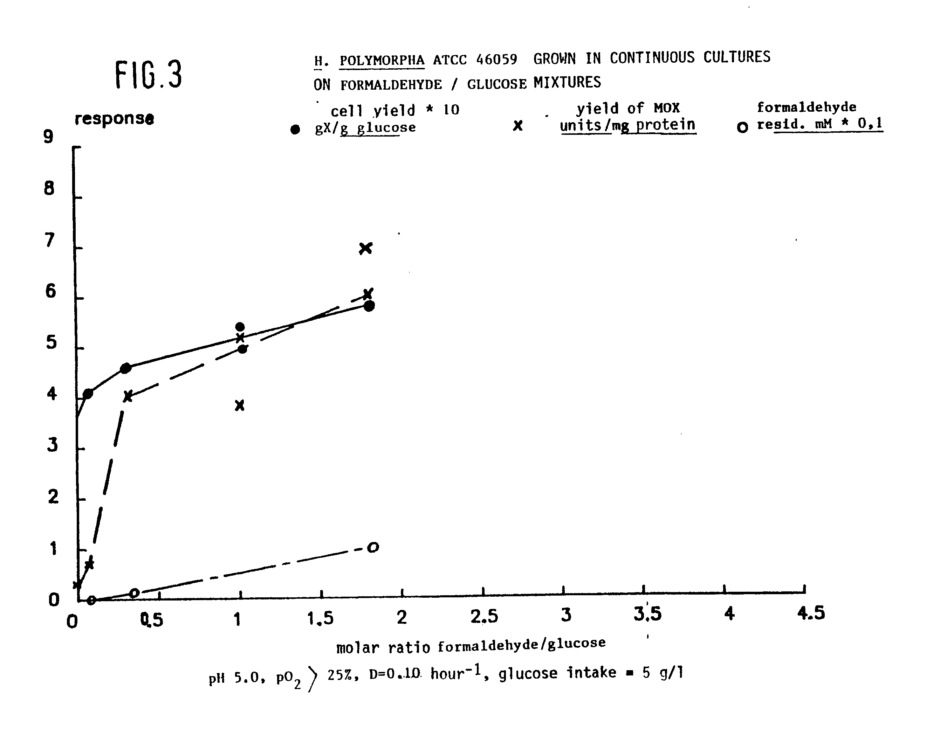

Hansenula polymorpha ATCC 46059 was grown on medium I with various formaldehyde/glucose mixtures at a dilution rate of 0.1 hour$^{-1}$. The values in stable state of specific MOX production and biomass yield on glucose are set out in Table B and Figure 3.

At a molar ratio of about 1.8 an optimal specific activity of MOX of 6 to 7 MOX units/mg protein was found.

In the cell lysates obtained from the above-mentioned cultures, no catalase activity could be detected.

## Example 3

### Isolation of MOX

Hansenula polymorpha ATCC 46059 was grown on medium II (Table A) at a dilution rate of 0.078 hour$^{-1}$. The ratio of formic acid to glucose was 2.65. The cell density was 39.9 gram biomass (dry weight)/litre.

Lysis of the cells with the aid of the zymolyase method with an incubation time of 1 to 3 hours at 37° C in a stirred cell suspension yielded no MOX activity.

Ultrasonic treatment of the cells gave a variable absolute yield of MOX and a low protein content compared with values of 40 to 50% protein/dry weight of the cells which are normally found in the wild-type strain CBS 4732.

Surprisingly, it was ascertained that the presence of oxygen and the reaction products of MOX with unknown cell components under aerobic conditions form the main cause of the MOX inactivation. The use of cell suspensions which have been flushed with nitrogen gas or the absence of excess catalase (more than 10 units/5 ml) in the cell suspension during the ultrasonic treatment led to an improvement of the absolute yield of MOX. See Table E.

The effect of the addition of catalase or of the flushing with nitrogen gas is greater at a formic acid/glucose ratio of 3.6.

A cell-free lysate was obtained by centrifuging for 15 minutes at 12,000 g.

The MOX activity in the cell-free lysate could be precipitated with a 65% saturated ammonium sulphate solution. The precipitate thus obtained was stable at room temperature. No catalase activity could be detected in the precipitate.

The precipitate obtained with ammonium sulphate can be applied for the uses described above.

## Example 4

Hansenula polymorpha ATCC 46059 was grown on medium II (pH 5.0; 37° C; pO$_2$ larger than 25% saturation) at a dilution rate of 0.078 hour$^{-1}$ in a 13-litre fermenter with a working volume of 9 litres medium. The cell density obtained in stable conditions was 39.9 g biomass (dry weight)/litre. The specific activity of the MOX, isolated with the aid of the method described in Example 3, was 2.1 MOX units/mg protein or 0.6 MOX units won per mg biomass (based on the dry weight).

The cells were freeze-dried for two days at 15°C under reduced pressure. The activity after renewed suspension of the freeze-dried sample in 0.05 M potassium phosphate buffer having a pH of 7.5 and ultrasonic treatment under aerobic conditions without catalase, was 0.6 MOX units per mg biomass (dry weight). After freeze-drying, the cells were stable at room temperature for at least one week.

The MOX activity was isolated from the freeze-dried cells by breaking open the cells in a mill with glass beads. The cell suspensions were prepared in an oxygen-free 0.05 M potassium phosphate buffer flushed with nitrogen, and stored under nitrogen. The suspension of lysed cells was centrifuged. The clear supernatant was precipitated by adding ammonium sulphate at a concentration of 65% saturation. The solution was centrifuged. The sediment obtained contained all MOX activity. The preparation was stable at room temperature and contained no catalase activity.

Table A

| Media used | | |
|---|---|---|
| | Medium I, based on Egli medium g $1^{-1}$ | Medium II, medium for high cell densities g $1^{-1}$ |
| Glucose | 9 | 90 |
| Formaldehyde or formic acid | 1 - 7 ml | 50 ml |
| $NH_4Cl$ | 7.63 | - |
| $KH_2PO_4$ | 2.81 | 6 |
| $MgSO_4.7H_2O$ | 0.59 | 4.5 |
| $CaCl_2.2H_2O$ | 0.055 | 0.6 |
| $FeSO_4.7H_2O$ | 0.0375 | - |
| $MnSO_4.H_2O$ | 0.014 | 0.01 |
| $ZnSO_4.7H_2O$ | 0.022 | 0.022 |
| $CuSO_4.5H_2O$ | 0.004 | 0.004 |
| $CoCl_2.6H_2O$ | 0.0045 | - |
| $Na_2MoO_4.2H_2O$ | 0.0026 | 0.0026 |
| $H_3BO_3$ | 0.004 | 0.004 |
| KJ | 0.0026 | 0.0026 |
| EDTA | 0.45 | - |
| Biotine | 0.00005 | 0.00005 |
| Thiamine HCl | 0.005 | 0.005 |
| Meso-inositol | 0.047 | 0.047 |
| Pyroxidine | 0.0012 | 0.0012 |
| D-pantothenic acid | 0.023 | 0.023 |
| NaCl | | 0.3 |
| $FeCl_3.6H_2O$ | | 0.035 |
| KCl | | 3 |
| $H_2SO_4$ (conc.) | | 0.03 ml |

Table B

| MOX production in cultures of H. polymorpha ATCC 46059, grown on mixtures of formaldehyde/glucose at a dilution rate of 0.1 hour$^{-1}$ | |
|---|---|
| Molar ratio formaldehyde / glucose | MOX activity MOX units / mg protein |
| 0.07 | 1.0 |
| 0.36 | 4.0 |
| 1.0 | 4-5 |
| 1.8 | 6-7 |

Table C

| MOX production in cultures of H. polymorpha ATCC 46059, grown on mixtures of formic acid/glucose at a dilution rate of 0.1 hour$^{-1}$ | |
| --- | --- |
| Molar ratio formic acid / glucose | MOX activity MOX units / mg protein |
| 0.0 | 0.05 |
| 0.52 | 0.025 |
| 1.6 | 1.5 |
| 2.6 | 2.7 |
| 2.9 | 3.5 |
| 3.6 | 2.5 |

Table D

| MOX production in cultures of H. polymorpha ATCC 46059, grown on a mixture with a formic acid/glucose ratio of 3.6 at different dilution rates | |
| --- | --- |
| Dilution rate hour$^{-1}$ | MOX activity MOX units / mg protein |
| 0.047 | 3.03 |
| 0.049 | 2.92 |
| 0.1 | 2.48 |
| 0.115 | 2.26 |
| 0.144 | 2.1 |
| 0.158 | 3.0 |
| 0.24 | 1.1 |

## Table E

Effect of flushing with nitrogen and presence of cata-
lase during ultrasonic treatment of cell suspensions on
MOX yield

| Treatment | | | MOX activity | Specific activity |
| --- | --- | --- | --- | --- |
| | | | MOX units / ml culture | MOX units / mg protein |
| Batch | Flushed with N$_2$ | Catalase during treatment | | |
| 1 | – | – | 15.8 | 1.7 |
| 1 | – | + | 21.5 | 2.07 |
| 1 | + | – | 20.0 | 1.92 |
| 2 | – | – | 0.47 | 2.2 |
| 2 | – | + | 2.95 | 2.2 |

Observation:

Batch 1 = cell density 39.9 g biomass / litre,
D = 0.078 hour$^{-1}$
ratio formic acid / glucose: 2.6

Batch 2 = cell density 5.08 g biomass / litre,
D = 0.1 hour$^{-1}$
ratio formic acid / glucose: 3.7.

In the Figures 1-3, the abbreviation RQ stands for respiration quotient.

## Claims

1. Process for the preparation of an oxidase or an oxidase-containing mixture or preparation by aerobic fermentation of a yeast under conditions in which the yeast produces oxidase, and, if desired, isolation from the yeast cells of the oxidase produced, characterized in that a catalase-negative mutant of a yeast is allowed to grow in a nutritive medium suitable for the yeast in the presence of at least one compound which induces the expression of the oxidase gene but is not a substrate for the oxidase.

2. Process according to claim 1, in which a catalase-negative mutant of a yeast of the genus Hansenula or of the genus Pichia is used, in particular of a yeast of the species Hansenula polymorpha or of the species Pichia pastoris, more in particular the catalase-negative mutant Hansenula polymorpha 55/11, ATCC 46059.

3. Process according to claim 1 or 2, in which a yeast is used that is modified with the aid of recombinant DNA techniques, or its progeny, in which the genetic information for the oxidase stands under control of a methanol oxidase (MOX) promoter, in particular the MOX-promoter of Hansenula polymorpha CBS 4732.

4. Process according to any one of the claims 1-3, in which methanol oxidase is produced as oxidase, in particular a methanol oxidase that has the same amino acid sequence as the known methanol oxidase of Hansenula polymorpha CBS 4732, or a derivative of this sequence obtained via enzyme engineering.

5. Process according to any one of the claims 1-4, in which formiate or formaldehyde is used as the compound which induces expression of the oxidase gene but is not a substrate for the oxidase.

6. Process according to any one of the claims 1-5, in which the yeast is cultured in a continuous fermentation.

7. Process according to any one of the claims 1-6, in which the yeast is cultured in the presence of a carbon source suitable for the chosen yeast and of a formiate, the molar ratio formiate : carbon source being 0.5:1 to 4.5:1, preferably being 2:1 to 3.5:1.

8. Process according to any one of the claims 1-6, in which the yeast is cultured in the presence of a carbon source suitable for the chosen yeast and of formaldehyde, the molar ratio formaldehyde : carbon source being 0.1 :1 to 3:1, preferably being 0.5:1 to 2.5:1.

9. Process according to any one of the claims 1-8, in which the yeast is cultured in a nutritive medium that contains glucose as carbon source.

10. Process according to any one of the claims 1-9, in which the oxidase produced is isolated from the yeast cells under oxygen-free conditions.

## Revendications

1. Procédé de préparation d'une oxydase ou d'un mélange ou préparation contenant une oxydase par fermentation aérobie d'une levure dans des conditions dans lesquelles la levure produit une oxydase et, le cas échéant, isolement de l'oxydase produite à partir des cellules de levure, caractérisé en ce qu'on laisse pousser un mutant à catalase négative d'une levure dans un milieu nutritif approprié pour la levure en présence d'au moins un composé qui provoque l'expression du gène oxydase mais n'est pas un substrat pour l'oxydase.

2. Procédé selon la revendication 1, dans lequel le mutant à catalase négative d'une levure du genre Hansenula ou du genre Pichia est utilisé, en particulier d'une levure du type Hansenula polymorpha ou du type Pichia pastoris, ou plus précisément encore le mutant à catalase négative Hansenula polymorpha55/11 ATCC 46059.

3. Procédé selon la revendication 1 ou 2, dans lequel on utilise une levure qui a été modifiée à l'aide de techniques d'ADN recombinant ou de son progène dans lequel l'information génétique pour l'oxydase reste sous contrôle d'une méthanol-oxydase (MOX) en qualité d'activant, en particulier l'activant MOX de Hansenula polymorpha CBS 4732.

4. Procédé selon l'une quelconque des revendications 1 à 3, dans lequel on produit la méthanol-oxydase comme oxydase, en particulier la méthanol-oxydase ayant la même séquence d'amino-acides que la méthanol-oxydase connue de Hansenula polymorpha CBS 4732, ou un dérivé de cette séquence qu'on obtient par génie enzymatique.

5. Procédé selon l'une quelconque des revendications 1 à 4, dans lequel on utilise le formiate ou le fomrladéhyde en qualité de composé induisant l'expression du gène oxydase mais qui n'est pas un substrat pour l'oxydase.

6. Procédé selon l'une quelconque des revendications 1 à 5, dans lequel on cultive la levure par fermentation continue.

7. Procédé selon l'une quelconque des revendications 1 à 6, dans lequel on cultive la levure en présence d'une source de carbone appropriée pour la levure choisie et d'un formiate, le rapport molaire formiate:source de carbone étant compris entre 0,5 : 1 et 4,5 : 1, de préférence entre 2:1 et 3,5:1.

8. Procédé selon l'une quelconque des revendications 1 à 6, dans lequel on cultive la levure en présence d'une source de carbone appropriée pour la levure choisie et de formaldéhyde, le rapport molaire formaldéhyde:source de carbone étant compris entre 0,1:1 et 3:1, de préférence entre 0,5:1 et 2,5:1.

9. Procédé selon l'une quelconque des revendications 1 à 8, dans lequel on cultive la levure dans un milieu nutritif contenant du glucose comme source de carbone.

10. Procédé selon l'une quelconque des revendications 1 à 9, dans lequel on isole l'oxydase produite des cellules de levure dans des conditions exemptes d'oxygène.

## Patentansprüche

1. Verfahren zur Herstellung einer Oxidase oder einer Oxidase enthaltenden Mischung oder zu deren Herstellung durch aerobe Fermentation einer Hefe unter Bedingungen, bei denen die Hefe Oxidase produziert, und, falls erwünscht, Isolierung der produzierten Oxidase von den Hefezellen, dadurch gekennzeichnet, daß ein katalase-negativer Mutant einer Hefe in einem für die Hefe geeigneten Nährmedium in Gegenwart von mindestens einer Verbindung, die die Expression des Oxidasegens induziert, aber kein Substrat für die Oxidase ist, wachsen kann.

2. Verfahren nach Anspruch 1, in dem ein katalase-negativer Mutant einer Hefe der Gattung Hansenula oder der Gattung Pichia, bevorzugt einer Hefe der Spezies Hansenula polymorpha oder der Spezies Pichia pastoris, besonders bevorzugt der katalase-negative Mutant Hansenula polymorpha 55/11, ATCC 46059, verwendet wird.

3. Verfahren nach Anspruch 1 oder 2, in dem eine Hefe, die mit Hilfe von DNA-Rekombinationstechniken modifiziert ist, oder ihre Nachkommen verwendet werden und in dem die genetische Information für die Oxidase unter der Kontrolle eines Methanoloxidase-Promotors (MOX), insbesondere des MOX-Promotors von Hansenula polymorpha CBS 4732, steht.

4. Verfahren nach irgendeinem der Ansprüche 1 bis 3, in dem Methanoloxidase als Oxidase, insbesondere eine Methanoloxidase, die dieselbe Aminosäuresequenz wie die bekannte Methanoloxidase von Hansenula polymorpha CBS 4732 aufweist, oder ein Abkömmling dieser Sequenz, der über Enzymverfahren erhalten wurde, produziert wird.

5. Verfahren nach irgendeinem der Ansprüche 1 bis 4, in dem Formiat oder Formaldehyd als die Verbindung verwendet wird, die die Expression des Oxidasegens induziert, aber die kein Substrat für die Oxidase ist.

6. Verfahren nach irgendeinem der Ansprüche 1 bis 5, in dem die Hefe mittels kontinuierlicher Fermentation kultiviert wird.

7. Verfahren nach irgendeinem der Ansprüche 1 bis 6, in dem die Hefe in Gegenwart einer Kohlenstoffquelle, die für die ausgewählte Hefe geeignet ist, und eines Formiats kultiviert wird, wobei das Molverhältnis Formiat : Kohlenstoffquelle 0,5 : 1 bis 4,5 : 1, vorzugsweise 2 : 1 bis 3,5 : 1, ist.

8. Verfahren nach irgendeinem der Ansprüche 1 bis 6, in dem die Hefe in Gegenwart einer Kohlenstoffquelle, die für die ausgewählte Hefe geeignet ist, und Formaldehyd kultiviert wird, wobei das Molverhältnis Formaldehyd : Kohlenstoffquelle 0,1 : 1 bis 3 : 1, vorzugsweise 0,5 : 1 bis 2,5 : 1, ist.

9. Verfahren nach irgendeinem der Ansprüche 1 bis 8, in dem die Hefe in einem Nährmedium kultiviert wird, das Glukose als Kohlenstoffquelle enthält.

10. Verfahren nach irgendeinem der Ansprüche 1 bis 9, in dem die produzierte Oxidase von den Hefezellen unter sauerstofffreien Bedingungen isoliert wird.

# FIG.1

cell yield * 0.1          yield of MOX units                RQ*    5
● gX/g glucose          X /mg protein                    o molCO$_2$/molO$_2$

response

molar ratio formic acid / glucose

pH 5.0, pO$_2$ ⟩ 25%, D=0.1 h$^{-1}$  glucose intake = 5 g/1, T=37°C

EP 0 244 920 B1

# FIG.2

H. POLYMORPHA ATCC 46059. GROWN ON A MIXTURE WITH A
FORMIC ACID / GLUCOSE RATIO 3.6

|  | MOX activity | yield * 10 | RQ* 5 | formic acid |
|---|---|---|---|---|
| x | units/mg protein | ● gX/g glucose | o $CO_2/O_2$ | ■ mM * 10 |

response

dilution rate, D    hour$^{-1}$

pH 5.0, T=30°C, $pO_2$ > 25% saturation

FIG.3

H. POLYMORPHA ATCC 46059 GROWN IN CONTINUOUS CULTURES
ON FORMALDEHYDE / GLUCOSE MIXTURES

cell yield * 10
● gX/g glucose

yield of MOX
X units/mg protein

formaldehyde
O resid. mM * 0,1

molar ratio formaldehyde/glucose

pH 5.0, pO$_2$ ⟩ 25%, D=0.10 hour$^{-1}$, glucose intake = 5 g/1

EP 0 244 920 B1